# EUROPÄISCHE PATENTSCHRIFT

(11) **EP 1 032 798 B1**
(45) Veröffentlichungstag und Bekanntmachung des Hinweises auf die Patenterteilung: **03.09.2003**
(21) Anmeldenummer: 98963479.5
(22) Anmeldetag: 18.11.1998
(51) Int. Cl.: F25J 3/02, C07C 7/00

(54) **VERFAHREN UND ANLAGE ZUM ABTRENNEN VON C2 - ODER C2+ - KOHLENWASSERSTOFFEN**
METHOD AND INSTALLATION FOR SEPARATING OFF C2 - OR C2+ - HYDROCARBONS
PROCEDE ET INSTALLATION POUR LA SEPARATION D'HYDROCARBURES C2 OU C2+

(30) Priorität: 27.11.1997 DE 19752703
(43) Veröffentlichungstag der Anmeldung: 06.09.2000
(73) Patentinhaber: Linde Aktiengesellschaft, 65189 Wiesbaden (DE)
(72) Erfinder: BAUER, Heinz, D-82067 Ebenhausen (DE)
(74) Vertreter: Kasseckert, Rainer
(86) Internationale Anmeldenummer: EP9807396
(87) Internationale Veröffentlichungsnummer: WO99028688

(56) Entgegenhaltungen:
- EP-A- 0 185 253
- WO-A-95/10011
- US-A- 4 401 450
- US-A- 4 596 588

## Beschreibung

Die Erfindung betrifft ein Verfahren zum Abtrennen von C₂- oder C₂₊-Kohlenwasserstoffen aus einem leichte Kohlenwasserstoffe und gegebenenfalls leichter als Methan siedende Komponenten enthaltenden Gasstrom, bei dem der unter erhöhtem Druck stehende Gasstrom abgekühlt, partiell kondensiert und in einer ersten Trennstufe in eine flüssige und eine gasförmige Fraktion getrennt und die flüssige Fraktion in einer zweiten Trennstufe durch Rektifikation in einen im wesentlichen C₂- oder C₂₊-Kohlenwasserstoffe enthaltenden Produktstrom und einen überwiegend leichtersiedende Komponenten enthaltenden Restgasstrom zeriegt wird, wobei die nach der partiellen Kondensation anfallende gasförmige Fraktion aus der ersten Trennstufe in einer dritten Trennstufe rektifikatorisch in eine flüssige und eine gasförmige Fraktion getrennt wird.

Femer betrifft die Erfindung eine Anlage zur Durchführung des erfindungsgemäßen Verfahrens mit mindestens einem Wärmetauscher zum Abkühlen und partiellen Kondensieren des Gasstromes, mit einer Trennvorrichtung zum Abtrennen des partiell kondensierten Teils des Gasstromes und mit einer Rektifizierkolonne zum Zerlegen des partiell kondensierten Teils des Gasstromes und mit einer Rückwaschkolonne.

Aus der EP-A 0 185 253 ist ein gattungsgemäßes Verfahren zur Gewinnung von C₂₊- oder von C₃₊-Kohlenwasserstoffen aus Gasgemischen, die im wesentlichen leichte Kohlenwasserstoffe und ggf. Wasserstoff oder Stickstoff enthalten, bekannt.

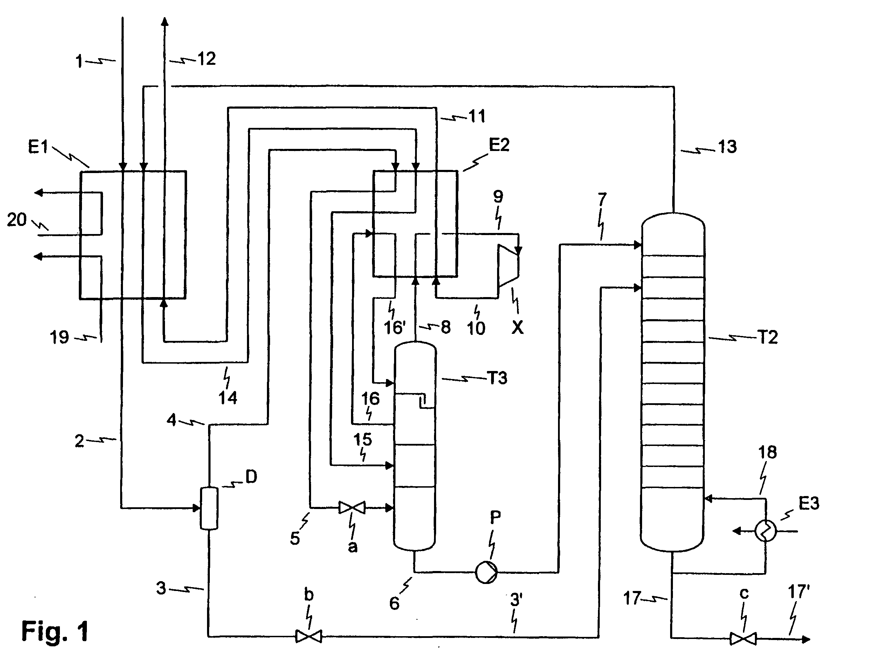

Das Verfahren gemäß der EP-A-0 185 253 ermöglicht eine sehr weitgehende Abtrennung der C₂₊- bzw. C₃₊-Kohlenwasserstoffe in der Rückwaschkolonne; siehe insbesondere die Figur 1 in Verbindung mit der entsprechenden Figurenbeschreibung. Dieser Effekt war im Falle der Verfahrensweise der EP-A-0 185 253 deshalb überraschend, weil die Kondensation der schweren, noch in der gasförmigen Fraktion enthaltenen Komponenten durch Inkontaktbringen mit einer leichteren Fraktion erreicht wurde. Bis dato wurden dagegen zum Auswaschen bestimmter Kohlenwasserstoffe aus einem Gas Kohlenwasserstoffe, die schwerer als die auszuwaschenden Komponenten sind, als Waschmittel eingesetzt.

Derartige Verfahren, wie sie z. B. in der genannten EP-A-0 185 253 beschrieben sind, dienen vor allem zur Abtrennung von Ethan oder von Propan aus Erdgasen oder anderen Gasen, bspw. Raffinerieabgasen. Außerdem eignen sich diese Verfahren zur Abtrennung von vergleichbaren ungesättigten Kohlenwasserstoffen, wie z. B. Ethylen oder Propylen, sofern diese Komponenten im zerlegenden Gasstrom enthalten sind, was bei den obengenannten Raffinerieabgasen der Fall sein kann.

Das Verfahren gemäß der EP-A 0 185 253 findet daher auch im sog. "kalten Zerlegungsteil" von Ethylen-Anlagen Verwendung. Ethylen läßt sich grundsätzlich auf zwei unterschiedlichen Wegen gewinnen. Gemäß dem klassischen Weg wird ein Spaltgas zunächst in einem sog. Front-End-Demethanizer einer C₁₋/C₂₊-Trennung unterworfen und die dabei gewonnene C₂₊-Fraktion anschließend rektifikatorisch in eine C₃₊-(Rest)Fraktion sowie in eine C₂-(Produkt)Fraktion aufgetrennt. Im Falle des zweiten Weges wird das Spaltgas in einem sog. Front-End-Deethanizer zunächst einer C₃₊/C₂₋-Trennung unterworfen. Die C₂₋-Fraktion, die in Spuren noch C₃₋-Kohlenwasserstoffe enthält, wird anschließend einer rektifikatorischen C₁₋/C₂-Trennung zugeführt und in dieser eine C₁₋-(Rest)Fraktion sowie eine C₂-(Produkt)Fraktion gewonnen.

Aufgabe der vorliegenden Erfindung ist die verbesserte Gewinnung der C₂- oder C₂₊-Fraktion, also ein Verfahren sowie eine Anlage zur Abtrennung von C₂- oder C₂₊-Kohlenwasserstoffen aus einem leichte Kohlenwasserstoffe und ggf. leichter als Methan siedende Komponenten enthaltenden Gasstrom anzugeben, bei dem bzw. bei der zum einen der Energieverbrauch der C₁/C₂-Trennung reduziert und zum anderen die Ausbeute an C₂- oder C₂₊-Kohlenwasserstoffen erhöht wird. Des weiteren liegt der vorliegenden Erfindung die Aufgabe zugrunde, ein Realisierung des erfindungsgemäßen Verfahrens in Rektifizierkolonnen, die aus preisgünstigeren Werkstoffen bestehen, zu ermöglichen.

Dies wird erfindungsgemäß dadurch erreicht, daß die in der dritten Trennstufe gewonnene flüssige Fraktion der ersten rektifikatorisch wirkenden Trennstufe als Rücklauf aufgegeben wird.

Die erfindungsgemäße Anlage zeichnet sich dadurch aus, daß der untere Bereich der Rückwaschkolonne mit dem oberen Bereich der Trennvorrichtung zum Abtrennen des partiell kondensierten Teils des Gasstromes verbunden ist und die Trennvorrichtung zum Abtrennen des partiell kondensierten Teils des Gasstromes als Strippkolonne ausgebildet ist.

Im Gegensatz zu der in der EP-A 0 185 253 beschriebenen Verfahrensweise bzw. Anlage wird die flüssige Fraktion aus der Rückwaschkolonne nicht der Rektifizierkolonne, sondern der ersten, rektifikatorisch wirkenden Trennkolonne zugeführt. Dadurch kann die flüssige Fraktion aus der Rückwaschkolonne in der ersten Trennkolonne, die anstelle des Abscheiders tritt, gegen das Einsatzgas gestrippt werden. Dies hat zur Folge, daß die Gesamtmenge der der Rektifizierkolonne zugeführten leichten Komponenten - also der C₁₋-Komponenten - verringert und dadurch die Trennaufgabe in der Rektifizierkolonne erleichtert wird.

Das aus der EP-A 0 185 253 bekannte Verfahren sowie das erfindungsgemäße Verfahren zur Abtrennung von C₂- oder C₂₊-Kohlenwasserstoffen und weitere Ausgestaltungen desselben seien im folgenden anhand der Figuren 1 bis 4 näher erläutert.

Hierbei zeigen:
- Figur 1:: Eine zum Stand der Technik zählende Verfahrensweise und Anlage gemäß der EP-A 0 185 253.
- Figur 2:: Eine erste Ausführungsform des erfindungsgemäßen Verfahrens, wobei die erste Trennkolonne keinen Zwischenrücklauf aufweist.
- Figur 3:: Eine zweite Ausführungsform des erfindungsgemäßen Verfahrens, wobei die erste Trennkolonne einen Zwischenrücklauf aufweist.
- Figur 4:: Eine dritte Ausführungsform des erfindungsgemäßen Verfahrens, wobei die Funktionen der ersten und dritten Trennkolonne in einer gemeinsamen Trennkolonne realisiert sind.

Der in der **Figur 1** über die Leitung 1 herangeführte Einsatzgasstrom, bestehend aus leichten Kohlenwasserstoffen und ggf. leichter als Methan siedenden Komponenten, ist bereits in geeigneter Weise für eine Tieftemperaturzerlegung vorbehandelt, d.h. es sind insbesondere solche Komponenten entfernt worden, die durch Ausfrieren Verlegungen hervorrufen oder unzulässige Korrosion verursachen würden. Dieser Gasstrom steht unter einem erhöhten Druck von vorzugsweise 25 bis 40 bar und weist eine Temperatur zwischen Umgebungstemperatur und -50 °C auf.

Der zu zerlegende Gasstrom wird in dem Wärmetauscher E1 soweit abgekühlt, daß der größte Teil der abzutrennenden und gewinnenden C₂- oder C₂₊-Kohlenwasserstoffe kondensiert. Der partiell kondensierte Strom wird aus dem Wärmetauscher E1 über Leitung 2 einem Abscheider D zugeführt und in diesem einer Phasentrennung unterzogen.

Die flüssige Fraktion bzw. das Kondensat aus dem Abscheider D wird über Leitung 3 einem Entspannungsventil b zugeführt, in diesem entspannt und über Leitung 3' der Rektifizierkolonne T2 in deren oberen Bereich zugeführt.

In der Rektifizierkolonne T2 wird die zugeführte flüssige Fraktion aus dem Abscheider D in eine C₂- oder C₂₊-Kohlenwasserstoff-Produktfraktion sowie in einen leichter siedende Anteile enthaltenden Restgasstrom zerlegt. Die C₂- oder C₂₊-Kohlenwasserstoff-Produktfraktion wird über Leitung 17 aus dem Sumpf der Rektifizierkolonne T2 abgezogen und dem Entspannungsventil c zugeführt, in diesem entspannt und über Leitung 17' als Produktstrom aus der Anlage abgeführt. Ein Teilstrom dieser C₂- oder C₂₊-Kohlenwasserstoff-Fraktion wird im Wärmetauscher E3 verdampft und über Leitung 18 wieder dem unteren Bereich der Rektifizierkolonne T2 zugeführt.

Der bereits erwähnte Restgasstrom wird der Rektifizierkolonne T2 am Kopf über Leitung 13 entnommen und dem Wärmetauscher E1 zugeführt. In diesem wird er abgekühlt, partiell kondensiert und anschließend über Leitung 14 einem weiteren Wärmetauscher E2 zugeführt. In diesem erfolgt eine weitere Abkühlung und partielle Kondensation, bevor er über Leitung 15 der Rückwaschkolonne T3 zugeführt wird.

Auch die aus dem Abscheider D über Leitung 4 abgezogene gasförmige Kopffraktion wird dem Wärmetauscher E2 zugeführt, in diesem abgekühlt und partiell kondensiert und anschließend über Leitung 5, in der ein Entspannungsventil a vorgesehen ist, der Rückwaschkolonne T3 zugeführt. Die in den beiden letztgenannten Strömen partiell kondensierten höher siedenden Komponenten werden aus der Rückwaschkolonne T3 über Leitung 6 abgezogen, mittels der Pumpe P auf den Druck der Rektifizierkolonne T2 gepumpt und dieser über Leitung 7 am Kopf als Rücklauf zugeführt.

Aus der Rückwaschkolonne T3 kann über die Leitung 16 ein Seitenstrom abgezogen, im Wärmetauscher E2 abgekühlt und partiell kondensiert und anschließend über die Leitung 16' der Rückwaschkolonne T3 als Rücklauf aufgegeben werden.

Am Kopf der Rückwaschkolonne T3 wird über Leitung 8 ein überwiegend Methan und leichter siedende Komponenten enthaltender Gasstrom abgezogen und im Wärmetauscher E2 angewärmt. Der angewärmte Gasstrom wird sodann über Leitung 9 einer Entspannungsturbine X, in der die benötigte Spitzenkälte des Prozesses erzeugt wird, zugeführt. Der abgekühlte Gasstrom wird über Leitung 10 wiederum dem Wärmetauscher E2 zugeführt und in diesem angewärmt. Anschließend wird er über Leitung 11 dem Wärmetauscher E1 zugeführt, in diesem gegen abzukühlende Verfahrensströme weiter angewärmt und über Leitung 12 aus dem Verfahren bzw. der Anlage - z. B. als Heizgasstrom - abgegeben.

Alternativ zu der in den Figuren 1 bis 4 dargestellten ein- oder mehrstufigen Entspannung mittels einer oder mehrerer Turbinen, könnte der über Leitung 8 aus der Rückwaschkolonne T3 abgezogene Gasstrom auch in einem, in den Figuren nicht dargestellten Entspannungsventil einer Joule-Thomson-Entspannung unterworfen werden.

Des weiteren kann alternativ oder zusätzlich zu der Entspannung mittels einer Turbine oder des Joule-Thomson-Effekts eine Fremdkältebereitstellung vorgesehen sein.

Die im Wärmetauscher E1 zur Abkühlung und partiellen Kondensation des Einsatzgasstromes sowie des Restgasstromes aus der Rektifizierkolonne benötigte Kälte wird durch bis zu drei externe Kältemittelkreisläufe bereitgestellt; in den Figuren 1 bis 3 sowie 4 sind der Übersichtlichkeit halber jeweils nur 2 Kältemittelkreisläufe 19 und 20 bzw. 3 Kältemittelkreisläufe 19, 20 und 29 dargestellt.

Femer ist der Übersichtlichkeit halber in der Figur 1 lediglich ein Abscheider D dargestellt. In der Realität werden jedoch zwei oder drei - selten mehr - Abscheider hintereinander geschaltet, wobei die Sumpffraktionen der einzelnen Abscheider der Rektifizierkolonne T2 zugeführt werden, während die Kopffraktionen - abgesehen von der Kopffraktion des dritten bzw. letzten Abscheiders - jeweils dem nachfolgenden Abscheider nach erfolgter Abkühlung und partieller Kondensation zugeführt werden.

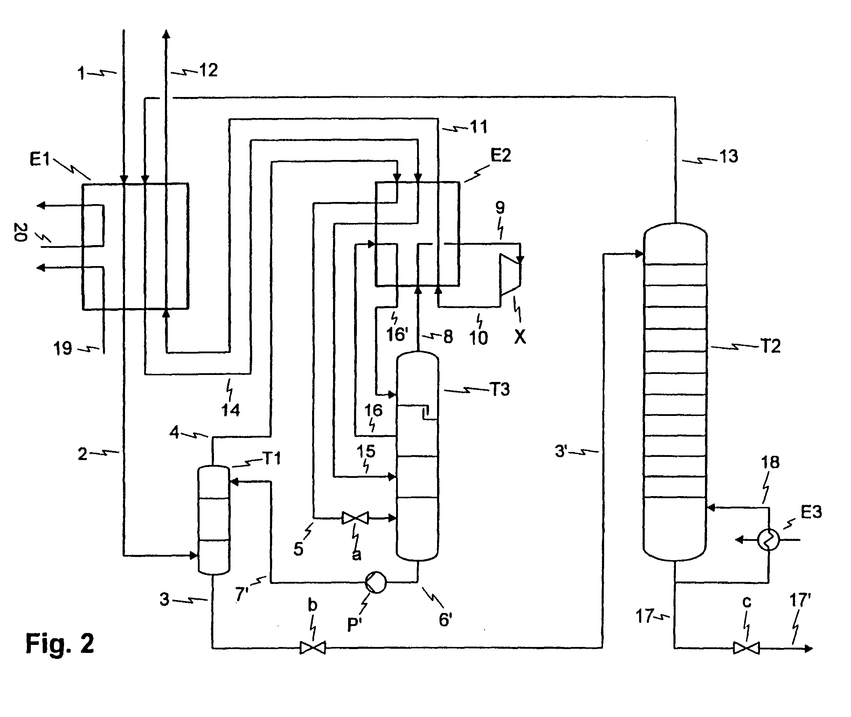

Die **Figur 2** zeigt, wie bereits erwähnt, eine erste Ausführungsform des erfindungsgemäßen Verfahrens, wobei die erste, rektifikatorisch wirkende Trennvorrichtung T1, die als Strippkolonne ausgebildet ist, keinen Zwischenrücklauf aufweist.

Der Einfachheit halber werden im folgenden nur die Unterschiede zwischen der zum Stand der Technik zählenden Verfahrensweise - wie sie in der Figur 1 dargestellt und anhand dieser erläutert ist - und dem erfindungsgemäßen Verfahren - wie es in den Figuren 2 bis 4 dargestellt ist - erläutert.

Der zu zerlegende Gasstrom, der im Wärmetauscher E1 abgekühlt und partiell kondensiert wird, wird über Leitung 2 nunmehr einer ersten, rektifikatorisch wirkenden Trennvorrichtung T1, die als Strippkolonne ausgebildet ist, zugeführt.

Entsprechend einer vorteilhaften Ausgestaltung des erfindungsgemäßen Verfahrens weist der unter erhöhtem Druck stehende, abgekühlte und partiell kondensierte Gasstrom vor seiner Zuführung in die Strippkolonne T1 eine Temperatur zwischen -100 und -40 °C, vorzugsweise zwischen -90 und -55 °C auf.

Während gemäß der Verfahrensweise der Figur 1 die im Sumpf der Rückwaschkolonne T3 anfallende Flüssigfraktion direkt der Rektifizierkolonne T2 zugeführt wird, wird diese Flüssigfraktion nunmehr über Leitung 6' abgezogen, mittels der Pumpe P' auf den Druck der Strippkolonne T1 gepumpt und dieser über Leitung 7' im Kopfbereich zugeführt.

Diese Verfahrensweise hat folgende Vorteile: Dadurch, daß die flüssige Sumpffraktion der Rückwaschkolonne T3 in der Strippkolonne T1 gegen das zu zerlegende Einsatzgas gestrippt wird, verringert sich die Gesamtmenge der der Rektifizierkolonne T2 zugeführten leichten Komponenten, also der Menge an Methan und leichter als Methan siedenden Komponenten. Mittels dieser Verfahrensführung wird die Trennaufgabe in der Rektifizierkolonne T2 erleichtert.

Des weiteren findet in der Strippkolonne T1 ein vorteilhafter Wärmeaustausch statt, da die flüssige Fraktion aus dem Sumpf der Rückwaschkolonne T3, die bisher einem vergleichsweise hohen und damit ungünstigen Temperatumiveau am Kopf der Rektifizierkolonne T2 zugeführt wurde - die Temperaturdifferenz zwischen der Kopftemperatur der Rektifizierkolonne T2 und der Temperatur der zugeführten Flüssigfraktion beträgt 36 °C -, entsprechend dem erfindungsgemäßen Verfahren der Strippkolonne T1 einem weitaus günstigeren Temperatumiveau zugeführt wird - die Temperaturdifferenz zwischen der Kopftemperatur der Strippkolonne T1 und der Temperatur der zugeführten Flüssigfraktion beträgt nunmehr lediglich 11 °C. Die erfindungsgemäße Verfahrensweise ermöglicht dadurch eine bessere Ausnutzung der mittels der Entspannungsturbine X erzeugten Spitzenkälte. Der Energieverbrauch der C₁/C₂-Trennung kann daher deutlich reduziert werden.

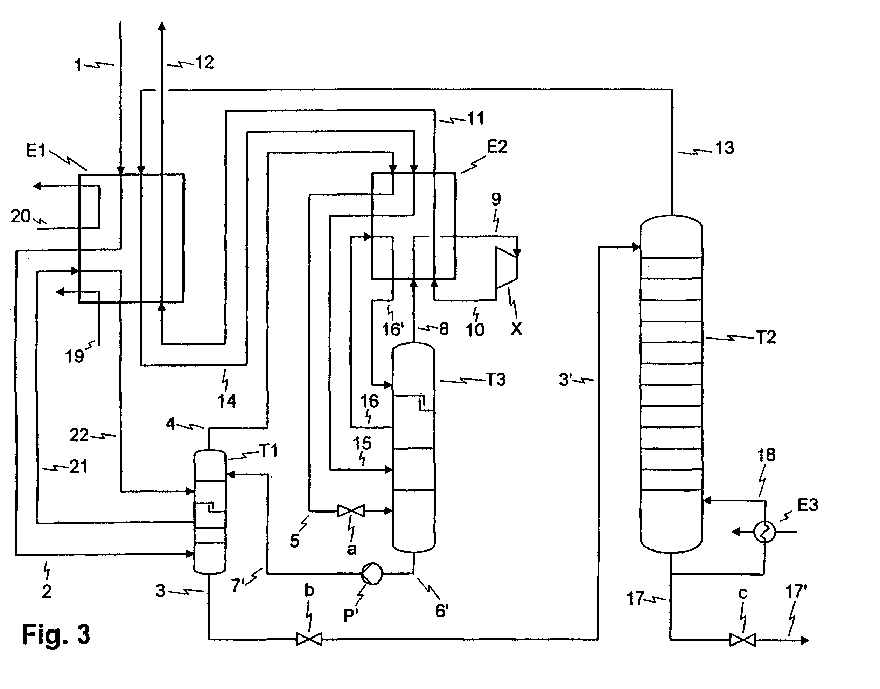

Die **Figur 3** zeigt eine zweite Ausführungsform des erfindungsgemäßen Verfahrens, bei der die Strippkolonne T1 einen Zwischenrücklauf aufweist Dazu wird über Leitung 21 aus dem unteren Bereich der Strippkolonne T1 ein Seitenstrom abgezogen, im Wärmetauscher E1 abgekühlt und partiell kondensiert und anschließend über Leitung 22 der Strippkolonne T1 als Zwischenrücklauf aufgegeben. Mittels dieser Ausführungsform des erfindungsgemäßen Verfahrens wird die Trennarbeit in der Strippkolonne T1 verbessert.

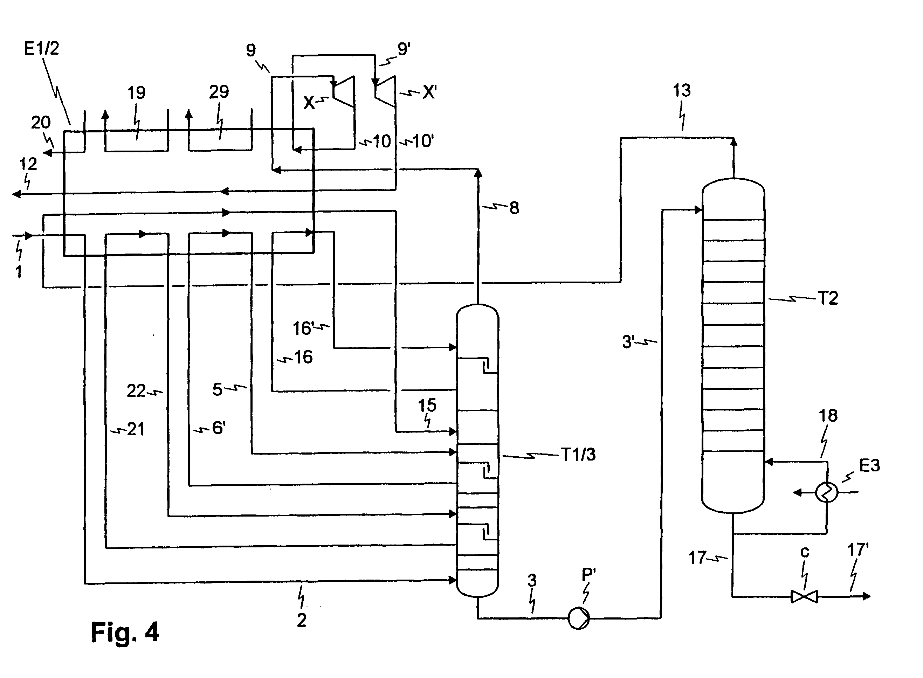

Eine weitere Ausführungsform des erfindungsgemäßen Verfahrens ist in der **Figur 4** dargestellt. Bei dieser Ausgestaltung des erfindungsgemäßen Verfahrens sind die Funktionen der ersten und dritten Trennkolonne T1 und T3 - wie sie in den Figuren 2 und 3 gezeigt sind - in einer gemeinsamen Trennkolonne T1/3 realisiert. Somit kann auf die die Trennkolonnen T1 und T3 verbindenden Leitungen 6' und 7'sowie die in ihnen angeordnete Pumpe P' verzichtet werden. Erforderlich ist nunmehr jedoch das Vorsehen einer Pumpe P' in den die Trennkolonnen T1/3 und T2 verbindenden Leitungen 3 und 3'.

Prinzipiell kann die in der Figur 4 dargestellte Trennkolonne T1/3 bzw. die in ihr stattfindende Rektifikation nicht nur in zwei separaten Trennkolonnen T1 und T3 - wie in den Figuren 2 und 3 gezeigt - sondern, abhängig von der Zahl der Zwischenkühlungsabzüge 21, 6' und 16, in mehrere separate Trennkolonnen "zerlegt" werden.

In der Figur 4 ist im Gegensatz zu den Figuren 1 bis 3 lediglich ein Wärmetauscher E1/2 dargestellt. Auch hier gilt, daß prinzipiell eine Vielzahl von separaten Wärmetauschem vorgesehen werden kann.

Der am Kopf der Trennkolonne T1/3 über Leitung 8 abgezogene, überwiegend Methan und leichter siedende Komponenten enthaltende Gasstrom wird nach einer Anwärmung im Wärmetauscher E1/2 über Leitung 9 einer ersten Entspannungsturbine X und nach erneuter Anwärmung im Wärmetauscher E1/2 über Leitung 9' einer zweiten Entspannungsturbine X', wobei in diesen Entspannungsturbinen die benötigte Prozessspitzenkälte erzeugt wird, zugeführt. Der abgekühlte Gasstrom wird anschließend über Leitung 10' wiederum dem Wärmetauscher E1/2 zugeführt, in diesem angewärmt und verläßt über Leitung 12 die Anlage.

Da bei dem erfindungsgemäßen Verfahren auf die tiefkalte Zuspeisung der flüssigen Sumpffraktion aus der Rückwaschkolonne T3 in die Rektifizierkolonne T2 verzichtet wird, kann die Rektifizierkolonne T2 aus kostengünstigeren Werkstoffen - Tieftemperaturstahl anstelle von hochlegiertem Stahl - hergestellt werden.

Der nachfolgenden Tabelle sind für ein konkretes Ausführungsbeispiel gemäß der **Figur 3** die entsprechenden Zusammensetzungen, Drücke, Temperaturen, etc zu entnehmen.

Während die Kopftemperatur der Rektifizierkolonne T2 bei dem Verfahren, wie es in der Figur 1 dargestellt ist, -58 °C beträgt, liegt sie bei der erfindungsgemäßen Verfahrensweise gemäß der Figur 3 bei -34 °C. Dies macht den Einsatz kostengünstiger Werkstoffe für die Rektifizierkolonne T2 möglich, da oberhalb einer Temperatur von -40 °C anstelle von hochlegiertem Stahl Tieftemperaturstahl verwendet werden kann.

| **Leitung** | **1** | **2** | **3** | **3'** | **4** | **5** | **6'** | **7'** | **8** | **9** | **10** |
|---|---|---|---|---|---|---|---|---|---|---|---|
| H₂ [Mol-%] | 44,3 | 44,3 | 1,9 | 2,0 | 68,9 | 68,9 | 1,5 | 1,5 | 75,0 | 75,0 | 75,0 |
| CO [Mol-%] | 0,0 | 0,0 | 0,0 | 0,0 | 0,1 | 0,1 | 0,0 | 0,0 | 0,1 | 0,1 | 0,1 |
| CH₄ [Mol-%] | 14,7 | 14,7 | 7,3 | 7,3 | 21,9 | 21,9 | 19,5 | 19,5 | 25,0 | 25,0 | 25,0 |
| C₂H₄ [Mol-%] | 28,5 | 28,5 | 64,2 | 64,2 | 8,4 | 8,4 | 67,6 | 67,6 | 0,0 | 0,0 | 0,0 |
| C₂H₆ [Mol-%] | 12,3 | 12,3 | 26,2 | 26,2 | 0,7 | 0,7 | 11,4 | 11,4 | 0,0 | 0,0 | 0,0 |
| C₃H₆ [Mol-%] | 0,0 | 0,0 | 0,2 | 0,2 | 0,0 | 0,0 | 0,0 | 0,0 | 0,0 | 0,0 | 0,0 |
| C₃H₈ [Mol-%] | 0,0 | 0,0 | 0,1 | 0,1 | 0,0 | 0,0 | 0,0 | 0,0 | 0,0 | 0,0 | 0,0 |
| Menge [kmol/h] | 5593 | 5593 | 2870 | 2870 | 3531 | 3531 | 808 | 808 | 3303 | 3303 | 3303 |
| Temp. [°C] | - 40 | - 53 | - 55 | - 55 | - 85 | - 96 | - 97 | - 97 | - 135 | - 128 | - 164 |
| Druck [bar] | 34,4 | 34,3 | 34,3 | 31,4 | 34,1 | 34,0 | 31,3 | 34,1 | 31,0 | 30,9 | 4,6 |

| **Leitung** | **11** | **12** | **13** | **14** | **15** | **16** | **16'** | **17** | **17'** | **21** | **22** |
|---|---|---|---|---|---|---|---|---|---|---|---|
| H₂ [Mol-%] | 75,0 | 75,0 | 9,8 | 9,8 | 9,8 | 61,4 | 61,4 | 0,0 | 0,0 | 56,2 | 56,2 |
| CO [Mol-%] | 0,1 | 0,1 | 0,0 | 0,0 | 0,0 | 0,1 | 0,1 | 0,0 | 0,0 | 0,1 | 0,1 |
| CH₄ [Mol-%] | 25,0 | 25,0 | 36,0 | 36,0 | 36,0 | 38,4 | 38,4 | 0,0 | 0,0 | 19,2 | 19,2 |
| C₂H₄ [Mol-%] | 0,0 | 0,0 | 42,8 | 42,8 | 42,8 | 0,0 | 0,0 | 69,6 | 69,6 | 22,1 | 22,1 |
| C₂H₆ [Mol-%] | 0,0 | 0,0 | 11,3 | 11,3 | 11,3 | 0,0 | 0,0 | 30,0 | 30,0 | 2,4 | 2,4 |
| C₃H₆ [Mol-%] | 0,0 | 0,0 | 0,0 | 0,0 | 0,0 | 0,0 | 0,0 | 0,3 | 0,3 | 0,0 | 0,0 |
| C₃H₈ [Mol-%] | 0,0 | 0,0 | 0,0 | 0,0 | 0,0 | 0,0 | 0,0 | 0,1 | 0,1 | 0,0 | 0,0 |
| Menge [kmol/h] | 3303 | 3303 | 579 | 579 | 579 | 4077 | 4077 | 2291 | 2291 | 4357 | 4357 |
| Temp. [°C] | - 88 | - 43 | - 44 | - 53 | - 82 | - 125 | - 135 | - 5 | - 49 | - 61 | - 82 |
| Druck [bar] | 4,4 | 4,2 | 31,4 | 31,3 | 31,2 | 31,1 | 31,3 | 31,8 | 9,2 | 34,2 | 34,1 |

## Patentansprüche

1. Verfahren zum Abtrennen von C₂- oder C₂₊-Kohlenwasserstoffen aus einem leichte Kohlenwasserstoffe und gegebenenfalls leichter als Methan siedende Komponenten enthaltenden Gasstrom (1, 2), bei dem der unter erhöhtem Druck stehende Gasstrom (1) abgekühlt (E1), partiell kondensiert und in einer ersten Trennstufe (T1) in eine flüssige (3, 3') und eine gasförmige Fraktion (4) getrennt und die flüssige Fraktion (3, 3') in einer zweiten Trennstufe (T2) durch Rektifikation in einen im wesentlichen C₂- oder C₂₊-Kohlenwasserstoffe enthaltenden Produktstrom (17, 17') und einen überwiegend leichtersiedende Komponenten enthaltenden Restgasstrom (13) zerlegt wird, wobei die nach der partiellen Kondensation anfallende gasförmige Fraktion (4) aus der ersten Trennstufe (T1) in einer dritten Trennstufe (T3) rektifikatorisch in eine flüssige (6') und eine gasförmige Fraktion (8) getrennt und die in der dritten Trennstufe (T3) gewonnene_flüssige Fraktion (6') der ersten rektifikatorisch wirkenden Trennstufe (T1) als Rücklauf aufgegeben wird (7').

2. Verfahren nach Anspruch 1, **dadurch gekennzeichnet, daß** die nach der partiellen Kondensation anfallende gasförmige Fraktion (4) aus der ersten Trennstufe (T1) vor ihrer Zuführung in die dritte Trennstufe (T3) abgekühlt und partiell kondensiert wird (E2).

3. Verfahren nach Anspruch 1 oder 2, **dadurch gekennzeichnet, daß** der Restgasstrom (13) aus der zweiten Trennstufe (T2) abgekühlt, partiell kondensiert (E1) und der dritten Trennstufe (T3) zugeführt wird.

4. Verfahren nach Anspruch 2 oder 3, **dadurch gekennzeichnet, daß** das Abkühlen der nach der partiellen Kondensation anfallenden gasförmigen Fraktion (4) und/oder des Restgasstromes (13) vor der Zuführung in die dritte Trennstufe (T3) im indirekten Wärmetausch (E2) mit Fremdkälte und/oder anzuwärmenden Verfahrensströmen, insbesondere im indirekten Wärmetausch mit dem mittels eines Ventils oder wenigstens einer Turbine (X, X') entspannten Restgasstrom (8) der dritten Trennstufe (T3), erfolgt.

5. Verfahren nach einem der vorhergehenden Ansprüche, **dadurch gekennzeichnet, daß** aus der ersten rektifikatorisch wirkenden Trennstufe (T1) wenigstens ein Teilstrom (21) abgezogen, abgekühlt, partiell kondensiert und der ersten Trennstufe (T1) als Zwischenrücklauf (22) zugeführt wird.

6. Verfahren nach einem der vorhergehenden Ansprüche, **dadurch gekennzeichnet, daß** die Temperatur des unter erhöhtem Druck stehenden, abgekühlten und partiell kondensierten Gasstromes (2) vor der Zuführung in die erste rektifikatorisch wirkenden Trennstufe (T1) zwischen -100 und -40 °C, vorzugsweise zwischen -90 und -55 °C beträgt.

7. Verfahren nach einem der vorhergehenden Ansprüche, **dadurch gekennzeichnet, daß** die Funktionen der ersten rektifikatorisch wirkenden Trennstufe (T1) und der dritten rektifikatorisch wirkenden Trennstufe (T3) in einer gemeinsamen Trennstufe (T1/3) realisiert werden.

8. Vorrichtung zum Abtrennen von C₂- oder C₂₊-Kohlenwasserstoffen aus einem leichte Kohlenwasserstoffe und gegebenenfalls leichter als Methan siedende Komponenten enthaltenden Gasstrom (1, 2), aufweisend
- mindestens einen Wärmetauscher (E1, E2, E1/2), in dem der unter erhöhtem Druck stehende Gasstrom (1) abgekühlt und partiell kondensiert wird,
- eine Strippkolonne (T1), in der der abgekühlte und partiell kondensierte Gasstrom (2) in eine flüssige (3, 3') und eine gasförmige Fraktion (4) getrennt wird,
- eine Rektifizierkolonne (T2), in der die in der Strippkolonne (T1) gewonnene Flüssigfraktion (3, 3') in einen im wesentlichen C₂- oder C₂₊-Kohlenwasserstoffe enthaltenden Produktstrom (17, 17') und einen überwiegend leichtersiedende Komponenten enthaltenden Restgasstrom (13) zerlegt wird,
- eine Rückwaschkolonne (T3), in der die in der Strippkolonne (T1) gewonnene Gasfraktion (4) rektifikatorisch in eine flüssige (6') und eine gasförmige Fraktion (8) getrennt wird,
- wobei der untere Bereich der Rückwaschkolonne (T3) mit dem oberen Bereich der Strippkolonne (T1) derart verbunden ist, daß die in der Rückwaschkolonne (T3) gewonnene Flüssigfraktion (6') der Strippkolonne (T1) als Rücklauf zuführbar ist.

9. Anlage nach Anspruch 8, **dadurch gekennzeichnet, daß** die Rückwaschkolonne (T3) und die Strippkolonne (T1) als eine Trennkolonne (T1/3) ausgebildet sind.

## Claims

1. Process for separating off C₂ or C₂₊ hydrocarbons from a gas stream (1, 2) comprising light hydrocarbons with or without components which boil at a lower temperature than methane, in which the gas stream (1), which is at elevated pressure, is cooled (E1), partially condensed and separated in a first separation stage (T1) into a liquid fraction (3, 3') and a gaseous fraction (4), and the liquid fraction (3, 3') is fractionated by rectification in a second separation column (T2) to form a product stream (17, 17') substantially comprising C₂ or C₂₊ hydrocarbons and a residual gas stream (13) predominantly comprising lower-boiling components, the gaseous fraction (4) from the first separation state (T1) which is produced downstream of the partial condensation being separated by rectification in a third separation stage (T3) into a liquid fraction (6') and a gaseous fraction (8), and the liquid fraction (6') obtained in the third separation stage (T3) being added (7') as reflux to the first separation stage (T1) which has a rectification action.

2. Process according to Claim 1, **characterized in that** the gaseous fraction (4) from the first separation stage (T1) produced downstream of the partial condensation is cooled and partially condensed (E2) before being fed into the third separation stage (T3).

3. Process according to Claim 1 or 2, **characterized in that** the residual gas stream (13) from the second separation stage (T2) is cooled, partially condensed (E1) and fed to the third separation stage (T3).

4. Process according to Claim 2 or 3, **characterized in that** the gaseous fraction (4) produced downstream of the partial condensation and/or the residual gas stream (13), before being fed into the third separation stage (T3), is cooled in indirect heat exchange (E2) with external refrigeration and/or process streams which are to be warmed, in particular in indirect heat exchange with the residual gas stream (8) of the third separation stage (T3), which has been expanded by means of a valve or at least one turbine (X, X').

5. Process according to one of the preceding claims, **characterized in that** at least one part stream (21) is extracted from the first separation stage (T1) which has a rectification action, is cooled, partially condensed and is fed to the first separation stage (T1) as intermediate reflux (22).

6. Process according to one of the preceding claims, **characterized in that** the temperature of the cooled and partially condensed gas stream (2), which is under elevated pressure, before it is fed into the first separation stage (T1) which has a rectification action, is between -100 and -40°C, preferably between -90 and -55°C.

7. Process according to one of the preceding claims, **characterized in that** the functions of the first separation stage (T1) which has a rectification action and the third separation stage (T3) which has a rectification action are implemented in a common separation stage (T1/3).

8. Installation for separating off C₂ or C₂₊ hydrocarbons from a gas stream (1, 2) comprising light hydrocarbons with or without components which boil at a lower temperature than methane, having
- at least one heat exchanger (E1, E2, E1/2), in which the gas stream (1), which is under elevated pressure, is cooled and partially condensed,
- a stripping column (T1), in which the cooled and partially condensed gas stream (2) is separated into a liquid fraction (3, 3') and a gaseous fraction (4),
- a rectification column (T2), in which the liquid fraction (3, 3') obtained in the stripping column (T1) is fractionated into a product stream (17, 17'), which substantially comprises C₂ or C₂₊ hydrocarbons, and a residual gas stream (13) which predominantly comprises lower-boiling components,
- a backwash column (T3), in which the gas fraction (4) obtained in the stripping column (T1) is separated by rectification into a liquid fraction (6') and a gaseous fraction (8),
- the lower region of the backwash column (T3) being connected to the upper region of the stripping column (T1) in such a manner that the liquid fraction (6') obtained in the backwash column (T3) can be fed to the stripping column (T1) as reflux.

9. Installation according to Claim 8, **characterized in that** the backwash column (T3) and the stripping column (T1) are designed as a separation column (T1/3).

## Revendications

1. Procédé pour la séparation d'hydrocarbures en C₂ ou C₂₊ d'un écoulement de gaz (1, 2) qui contient des hydrocarbures légers et éventuellement des composants plus volatils que le méthane, dans lequel l'écoulement de gaz (1) sous pression élevée est refroidi (E1), partiellement condensé et séparé dans une première étape de séparation (T1) en une fraction liquide (3, 3') et une fraction gazeuse (4), tandis que dans une deuxième étape de séparation, la fraction liquide (3, 3') est convertie (T2) par rectification en un écoulement de produits (17, 17') qui contient essentiellement des hydrocarbures en C₂ ou C₂₊ et en un écoulement (13) de gaz résiduels qui contient principalement des composants plus volatils, et dans une troisième étape de séparation (T3), la fraction gazeuse (4) de la première étape de séparation (T1) est séparée par rectification en une fraction liquide (6') et une fraction gazeuse (8), la fraction liquide (6') obtenue dans la troisième étape de séparation (T3) étant renvoyée pour alimenter dans la première de séparation (T1) qui travaille par rectification.

2. Procédé selon la revendication 1, **caractérisé en ce que** la fraction gazeuse (4) qui provient de la première étape de séparation (T1) est refroidie et partiellement condensée (E2) avant son envoi dans la troisième étape de séparation (T3).

3. Procédé selon les revendications 1 ou 2, **caractérisé en ce que** l'écoulement (13) de gaz résiduels qui provient de la deuxième étape de séparation (T2) est refroidi, partiellement condensé (E1) et envoyé dans la troisième étape de séparation (T3).

4. Procédé selon les revendications 2 ou 3, **caractérisé en ce que** le refroidissement de la fraction (4) obtenue par condensation partielle et/ou de l'écoulement (13) de gaz résiduels s'effectue avant l'envoi dans la troisième étape de séparation (T3) par échange de chaleur indirect (E2) avec du froid extérieur et/ou des écoulements du procédé qui doivent chauffés et en particulier par échange de chaleur indirect avec l'écoulement de gaz résiduels (8) de la troisième étape de séparation (T3) qui est détendu au moyen d'une soupape ou d'au moins une turbine (X, X').

5. Procédé selon au moins l'une des revendications précédentes, **caractérisé en ce qu'**au moins un écoulement partiel (21) est soutiré de la première étape de séparation (T1) qui travaille par rectification, est refroidi et condensé et renvoyé dans la première étape de séparation (T1) en tant qu'écoulement de recirculation (22).

6. Procédé selon au moins l'une des revendications précédentes, **caractérisé en ce que** la température de l'écoulement (2) de gaz sous pression, refroidi et partiellement condensé est comprise entre -100°C et -40°C, de préférence entre -90 et -55°C avant son renvoi dans la première étape de séparation (T1) qui travaille par rectification.

7. Procédé selon au moins l'une des revendications précédentes, **caractérisé en ce que** les fonctions de la première étape de séparation (T1) qui travaille par rectification et de la troisième étape de séparation (T3) qui travaille par rectification sont réalisées dans une étape commune de séparation (T1/3).

8. Dispositif pour la séparation d'hydrocarbures en C₂ ou C₂₊ d'un écoulement de gaz (1, 2) qui contient des hydrocarbures légers et éventuellement des composants plus volatils que le méthane, qui contient :
- au moins un échangeur de chaleur (E1, E2, E1/2) dans lequel l'écoulement (1) de gaz sous pression est refroidi et partiellement condensé,
- une colonne de séparation (T1) dans laquelle l'écoulement (2) de gaz sous pression refroidi et partiellement condensé est séparé en une fraction liquide (3, 3') et en une fraction gazeuse (4),
- une colonne de rectification (T2) dans laquelle la fraction liquide (3, 3') obtenue dans la colonne de séparation (T1) est décomposée en un écoulement de produits (17, 17') qui contient essentiellement des hydrocarbures en C₂ ou C₂₊ et en un écoulement (13) de gaz résiduels qui contient principalement des composants plus volatils,
- une colonne de lavage (T3) dans laquelle la fraction gazeuse (4) obtenue dans la colonne de séparation (T1) est séparée par rectification en une fraction liquide (6') et une fraction gazeuse (8),
- la partie inférieure de la colonne de lavage (T3) étant reliée à la partie supérieure de la colonne de séparation (T1) de telle sorte que la fraction liquide (6') obtenue dans la colonne de lavage (T3) puisse être renvoyée comme recirculation dans la colonne de séparation (T1).

9. Installation selon la revendication 8, **caractérisée en ce que** la colonne de lavage (T3) et la colonne de séparation (T1) sont configurées comme colonne de séparation (T1/3).
